Europäisches Patentamt

European Patent Office

Office européen des brevets

(19)

(11) Numéro de publication : **0 106 732 B1**

(12) **FASCICULE DE BREVET EUROPÉEN**

(45) Date de publication du fascicule du brevet :
09.09.87

(21) Numéro de dépôt : 83401795.6

(22) Date de dépôt : 14.09.83

(51) Int. Cl.⁴ : **G 01 N 3/36**, G 01 N 33/44

(54) **Appareillage pour le contrôle de la résistance à la pliure de feuilles thermoplastiques.**

(30) Priorité : 23.09.82 FR 8216011

(43) Date de publication de la demande :
25.04.84 Bulletin 84/17

(45) Mention de la délivrance du brevet :
09.09.87 Bulletin 87/37

(84) Etats contractants désignés :
BE DE GB IT LU NL

(56) Documents cités :
DE-A- 2 745 182
US-A- 2 426 583
US-A- 3 906 784
SOVIET MATERIALS SCIENCE, vol. 10, no. 2, mars-avril 1974, Plenum Publishing corporation, New York (US), V.I. ARONS et al.: "Instrument for determining the susceptibility of metals to hydrogen embrittlement", pages 206-208
MECANIQUE, MATERIAUX, ELECTRICITE, no. 276, décembre 1972, Paris (FR), P. FINK: "Une nouvelle methode pour la determination de la rigidite", pages 19-27

(73) Titulaire : **SOCIETE CHIMIQUE DES CHARBONNAGES SA**
**Tour Aurore Cédex 5**
**F-92080 Paris la Défense 2 (FR)**

(72) Inventeur : **Wirth, René**
**4, rue Bertnelot**
**F-62300 Lens (FR)**

(74) Mandataire : **Rieux, Michel et al**
**C d F Chimie S.A. Service Propriété Industrielle Tour Aurore - Cédex no. 5**
**F-92080 Paris la Défense 2 (FR)**

## Description

La présente invention concerne un appareillage pour le contrôle de la résistance à la pliure de feuilles de thermoplastique. Elle a plus particulièrement pour objet un appareillage convenant pour contrôler la résistance à la pliure alternée de feuilles de polystyrène.

Le polystyrène est une résine thermoplastique, qui, grâce à la facilité avec laquelle il peut être travaillé, est largement utilisé dans l'industrie des plastiques. Les transformateurs de matières plastiques le soumettent généralement à des traitements par extrusion, thermoformage en particulier, qui permettent de fabriquer des produits finis à base de polystyrène. On a constaté que la facilité d'extrusion ou de thermoformage de polystyrène allait de pair avec une caractéristique « mécanique » qui est la résistance à la pliure alternée d'une feuille de polystyrène.

Cette résistance à la pliure alternée était, jusqu'à présent évaluée empiriquement. Sa mesure consistait à plier en deux alternativement dans chaque sens une feuille de polystyrène jusqu'à ce qu'on puisse visualiser l'apparition de fissures à l'endroit où la feuille était soumise à la pliure. Le nombre plus ou moins élevé d'aller-retours de la feuille de polystyrène jusqu'à apparition des fissures permettait de se faire une idée approximative de la valeur de la résistance à la pliure alternée et ainsi d'évaluer les possibilités d'application de la résine testée.

Il va de soi que la détermination de la résistance à la pliure alternée, selon cette méthode est manuelle et empirique et, par conséquent, n'est pas rigoureuse et manque de fiabilité. En effet du polystyrène pour lequel le test à la résistance à la pliure alternée réalisé manuellement est bon, peut avoir en réalité du thermoformage profond un comportement si mauvais que la feuille de polystyrène obtenue peut être perforée. De plus ce test réalisé manuellement et qui permet de considérer le polystyrène testé comme bon, conduit, en fait à un thermoplastique qui extrudé, ne convient pas pour toutes les applications ultérieures. Dans ces conditions, des quantités importantes de résines considérées comme bonnes ne sont plus utilisables pour les applications ultérieures, ce qui risque de provoquer des pertes énormes.

La mise au point d'une méthode de mesure rigoureuse et fiable semble donc indispensable dans l'industrie des thermoplastiques, du polystyrène en particulier, ne serait-ce que pour éviter les pertes de résines dues à une mauvaise évaluation de la résistance à la pliure alternée.

On a maintenant trouvé un appareillage qui permet de contrôler de façon fiable la résistance à la pliure de feuilles de polystyrène. Le schéma ci-joint donné à titre d'illustration permet de situer les différents organes de l'appareillage objet de l'invention.

La présente invention concerne un appareillage comme décrit dans la revendication 1.

La mise en œuvre de l'appareillage pneumatique selon l'invention permet de faire varier en fonction de la feuille thermoplastique à contrôler, deux paramètres à savoir la vitesse et l'angle de rotation du disque. Seul un dispositif pneumatique permet une telle latitude : en effet, il n'est pas possible par exemple en utilisant un dispositif électrique tel que décrit dans Soviet Materials Science, vol. 10, N° 2, mars-avril 1974, Plenum Publishing Corporation pages 206 à 208 d'avoir une vitesse linéaire régulière du disque rotatif.

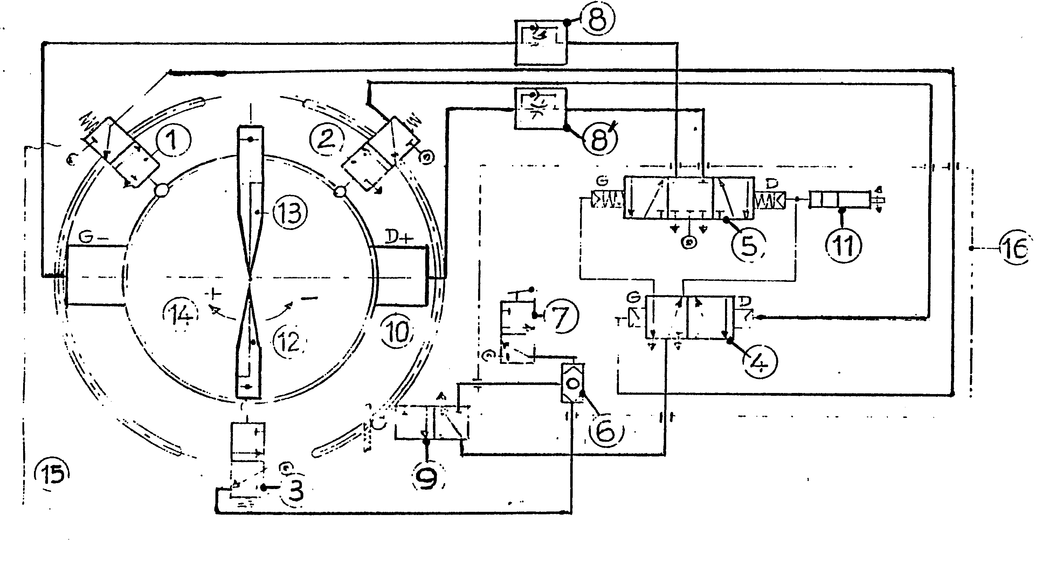

Le fonctionnement de l'appareillage objet de l'invention peut être mieux compris par la description détaillée suivante accompagnée par la lecture de la figure unique donnée à titre d'illustration.

L'appareil est constitué des éléments suivants :
une unité rotative (10)
une cloison fixe verticale (15)
deux distributeurs ou butées pneumatiques (1) et (2) disposés concentriquement à l'unité rotative (10) et s'appuyant respectivement sur des supports réglables (supports non représentés).
un autre distributeur ou butée pneumatique (3) disposé aussi concentriquement à l'unité rotative (10) non réglable et fixé sur la cloison (15)
deux autres distributeurs (4) et (5)
un sélecteur (6) alimenté par le distributeur (7) ou (3)
deux limiteurs de débit (8) et (8')
un distributeur (9) qui est le distributeur de sécurité.

L'unité rotative (10) est constituée d'un disque mobile (14) dont le déplacement alternatif est assuré par une roue dentée montée sur une crémaillère qui constitue l'axe d'un piston (dispositif non représenté). Sur le disque mobile (14) disposé sur une cloison verticale (15) est fixée une mâchoire (12) qui se déplace donc avec le disque ; une autre mâchoire fixe (13) est fixée à l'extérieur du disque mobile : c'est entre ces deux mâchoires que l'on fixe la feuille de thermoplastique dont on veut évaluer la résistance à la pliure.

Les distributeurs pneumatiques (1) et (2) sont actionnés en fin de course par la mâchoire mobile (12) munie d'une came : cette action est transmise par l'intermédiaire des deux butées réglables (1) et (2), ce qui se solde suivant qu'il y a ouverture ou fermeture des distributeurs par la création d'un mouvement de rotation alternatif du disque mobile (14).

Le distributeur pneumatique (3) permet l'arrêt en position d'alignement des deux mâchoires (12) et (13) après arrêt du distributeur manuel (7). Le distributeur (4) alimente le distributeur (5) après transmission de l'action de la mâchoire (12). Le distributeur (9) est un distributeur qui permet d'arrêter tout l'appareillage en cas d'accident. Le distributeur (7) est à fonctionnement manuel. Il permet de faire démarrer ou d'arrêter l'unité.

Un sélecteur (6) assure le maintien de la pression dans l'appareillage en supprimant l'alimentation par le distributeur (7) du distributeur (5) mais en la maintenant par le distributeur (3) : les mâchoires (12) et (13) sont .alors en position d'alignement. Les deux limiteurs de débit (8) et (8') servent à régler les débits d'air dans l'appareillage. On a représenté en (11) un compteur qui permet de comptabiliser le nombre de tours du disque mobile jusqu'à apparition de craquelures des feuilles de thermoplastique.

L'appareillage fonctionne de la façon suivante : il est alimenté par de l'air comprimé provenant d'une source (non représenté sur le schéma). Sur la figure unique, on a représenté les deux circuits d'air comprimé en traits pleins et en traits discontinus :

les traits pleins représentent ce qu'on appelle le circuit « d'air de puissance », c'est-à-dire le circuit d'air qui agit par l'intermédiaire des organes précédemment décrits sur l'unité rotative (10) et qui provoque la rotation du disque (14)

les traits discontinus représentent le circuit d'air secondaire que l'on appelle circuit « d'air de pilotage » qui actionne les différents organes pneumatiques de l'appareillage en ouvrant ou en fermant les orifices de ces organes en particulier des distributeurs pneumatiques.

La feuille de thermoplastique dont on veut évaluer la résistance à la pliure est serrée entre les mâchoires (12) et (13) qui sont à l'arrêt, face à face. Le distributeur étant en position arrêt c'est-à-dire que le circuit « d'Air de puissance » vers l'unité rotative (10) est coupé.

Lorsque l'appareillage fonctionne, le distributeur (7) alimente en cascade le sélecteur (6) qui actionne le distributeur (9), cette alimentation actionnant de ce fait « l'air de pilotage » D du distributeur (5). Dans cette position « l'air de puissance » circule vers l'unité rotative (10) par le côté (G) en passant par le limiteur de débit (8), et actionne ainsi la crémaillère de l'unité mobile ce qui provoque l'entraînement du disque mobile (14). L'air de la chambre de l'unité rotative (10) côté D s'échappe par le distributeur (5). En fin de course la came de la mâchoire (12) actionne le distributeur (2) et libère de ce fait la source d'air qui alimente en « air de pilotage » le distributeur (4) par le côté G. Le distributeur (7) étant toujours alimenté en air, il actionne le distributeur (5) côté G l'air passant par le sélecteur (6) et le distributeur (9). Dans cette position « l'air de puissance » circule vers l'unité rotative (10) par le côté D en passant par le limiteur de débit (8') et actionne ainsi la crémaillère du disque mobile (14).

Chaque aller-retour du disque mobile est comptabilisé à l'aide du compte-tours (11). Le mouvement de rotation alternatif provoque sur la feuille thermoplastique des fissures : on relève le nombre de tours jusqu'à apparition de la première fissure.

La comparaison du nombre de tours nécessaires pour l'apparition de fissures obtenu à vitesse constante et avec un angle de rotation constant permet d'étalonner les thermoplastiques.

**Revendication**

Appareillage adapté pour le contrôle de la résistance à la pliure de feuilles de thermoplastique comportant un dispositif rotatif et des moyens d'entraînement en rotation de ce dispositif caractérisé en ce que le dispositif rotatif est constitué d'une unité rotative (10) retransmettant un mouvement de rotation angulaire réglable de vitesse variable à un disque mobile (14) disposé sur une cloison verticale (15) le disque et la cloison verticale étant co-planaires et dans un même plan vertical, le disque mobile portant perpendiculairement à son plan une mâchoire (12) munie sur sa partie inférieure, d'une came intégrée, la cloison verticale (15) portant une mâchoire fixe (13) les deux mâchoires étant, à l'arrêt du disque, symétriques par rapport à l'axe de rotation du dispositif rotatif et situées dans un même plan vertical, la mâchoire fixe étant située au-dessus de l'axe de rotation, et une feuille de thermoplastique étant fixée entre les deux mâchoires (12, 13), de deux butées pneumatiques fixes 1 et 2 sur la cloison verticale (15) réglables entre elles angulairement d'un angle compris entre 130 et 300° par déplacement circulaire autour du disque mobile (14), d'une butée pneumatique (3) fixée à demeure sur la cloison (15) sous la mâchoire (12) dans son prolongement à l'arrêt du disque et à la même distance de l'axe de rotation du disque que les butées réglables (1, 2), · un mouvement de rotation alternatif du disque mobile étant obtenu par l'intermédiaire des 2 butées pneumatiques réglables (1) et (2) qui alimentent en cascade des distributeurs pneumatiques (4) et (5), cette alimentation provoquant le déplacement et une inversion du sens de rotation du disque mobile (14) sous l'action de la came intégrée de la mâchoire (12).

**Claim**

Apparatus adapted to control resistance to folding of thermoplastic sheets comprising a rotary device and means for driving the device in rotation, characterised in that the rotary device is comprised of a rotary unit (10) retransmitting an adjustable angular rotary movement with adjustable speed to a moving disc (14) disposed on a vertical partition (15) the disc and the vertical partition being co-planar and in the same vertical plane, the moving disc supporting perpendicular to its plane a jaw (12) fitted on its lower part with an integral cam, the vertical partition (15) bearing a secured jaw (13) the two jaws being, when the disc is stationary, symmetrical relative to the axis of rotation of the rotary device and located in the same vertical plane, the fixed jaw being located above the axis the rotation, and a thermoplastic sheet being secured between the two jaws (12) and (13), two fixed pneumatic stops 1 and 2 on the partition (15) which are adjustable with respect to one another angularly by an angle of between 130 and 130° by circular displacement about the moving disc (14), a pneumatic stop secured so as to rest on the partition (15), below the jaw (12) in its extension when the disc is stationary and at the same distance from the axis of rotation of the disc as the adjustable stops (1) and (2), an alternating rotary movement of the moving disc being obtained by means of 2 adjustable pneumatic stops (1) and (2) which supply the pneumatic distributors (4) and (5) in series, this supply causing the displacement and reversal of the direction of rotation of the moving disc under the action of the integral cam of the jaw (12).

**Patentanspruch**

Vorrichtung zum Kontrollieren der Beigefestigkeit von thermoplastischem Flachmaterial, mit einer drehbaren Vorrichtung und einem Drehantrieb für diese Vorrichtung, dadurch gekennzeichnet, daß die Drehvorrichtung eine sich drehende Einheit (10) aufweist, die eine einstellbare Drehwinkelbewegung mit einstellbarer Geschwindigkeit an eine bewegliche Scheibe (14) weitergibt, die auf einer vertikalen Zwischenwand. (15) angeordnet ist, wobei die Scheibe und die vertikale Zwischenwand in derselben vertikalen Ebene ko-planar sind und die bewegliche Scheibe senkrecht zu ihrer Ebene eine Klemme (12) aufweist, die an ihrem unteren Teil eine integrierte Nockenscheibe aufweist, wobei die vertikale Zwischenwand (15) eine feste Klemme (13) aufweist, wobei die beiden Klemmen bei stehender Scheibe symmetrisch bezüglich der Drehachse der drehbaren Vorrichtung und in derselben Vertikalebene angeordnet sind, wobei die ortsfeste Klemme oberhalb der Drehachse angeordnet ist und eine Platte aus thermoplastischem Material zwischen den beiden Klemmen (12) und (13) befestigt ist, wobei zwei pneumatisch befestigte Anschläge (1) und (2) auf der vertikalen Zwischenwand (15) untereinander winkelmäßig mit einem Winkel zwischen 130 und 300° durch eine kreisförmige Bewegung um die bewegliche Scheibe (14) herum einstellbar sind, wobei ein pneumatischer Anschlag (3) fest auf der Zwischenwand (15) unter der Klemme (12) in dessen Verlängerung bei stehender Schiebe befestigt ist und den gleichen Abstand zur Drehachse der Scheibe hat wie die einstellbaren Anschläge (1) und (2), wobei eine Drehbewegung mit wechselndem Drehsinn der beweglichen Scheibe mit Hilfe der beiden pneumatischen einstellbaren Anschläge (1) und 2 erhalten wird, die abwechselnd mit pneumatischen Verteilern (4) und (5) gespeist werden, wobei diese Speisung die Bewegung und Umkehrung des Drehsinns der beweglichen Scheibe (14) unter Einwirkung der in der Klemme (12) integrierten Kurvenscheibe, bewirken.